# FASCICULE DE BREVET EUROPEEN

(11) **EP 1 496 800 B1**
(45) Date de publication et mention de la délivrance du brevet: **23.01.2013**
(21) Numéro de dépôt: 03745824.7
(22) Date de dépôt: 04.04.2003
(51) Int. Cl.: A61B 6/00

(54) **SYSTEM D AIDE A LA NAVIGATION EN TEMPS REEL POUR DISPOSITIF DE RADIOGRAPHIE**
ECHTZEIT-NAVIGATIONSHILFESYSTEM UND RADIOGRAPHIE-SYSTEM
REAL-TIME NAVIGATIONAL AID SYSTEM FOR RADIOGRAPHY

(30) Priorité: 05.04.2002 FR 0204296
(43) Date de publication de la demande: 19.01.2005
(73) Titulaire: Vallée, Jean-Noel, 75005 Paris (FR); Nioche, Christophe, 75012 Paris (FR); Sabbah, Patrick, 94160 Saint-Mande (FR)
(72) Inventeur: Vallée, Jean-Noel, 75005 Paris (FR); Nioche, Christophe, 75012 Paris (FR); Sabbah, Patrick, 94160 Saint-Mande (FR)
(74) Mandataire: Regimbeau
(86) Numéro de dépôt international: PCT/FR2003/001075
(87) Numéro de publication internationale: WO 2003/084380

(56) Documents cités:
- DE-A- 10 047 314
- US-A- 5 274 551
- US-A- 5 960 054
- US-A- 6 075 837
- US-B1- 6 196 715

## Description

L'invention concerne un système d'aide au guidage sous imagerie, d'instruments et de matériels au sein d'une région d'intérêt.

Un volume de la région d'intérêt est un volume objet sans caractère limitatif de représentation concernant la forme externe de l'objet ou la forme interne de l'objet, obtenu à partir de toute technique d'imagerie permettant de produire de tels volumes.

Des images actives temps réel sont des images animées temps réel obtenues par toute technique d'imagerie capable de produire ces images.

Des instruments et matériels sont des instrumentations visualisables par la technique d'imagerie produisant les images actives temps réel.

Actuellement, les procédures de radiologie interventionnelle assistées sous imagerie par angiographie, par exemple, pour l'investigation ou le traitement d'une région anatomique d'intérêt sont actuellement réalisées à l'aide d'un système d'aide au radioguidage basé sur des images de référence planes selon 2 modes possibles de radioscopie :
- La radioscopie en mode superposition consiste à superposer une image plane de référence, soustraite ou non soustraite, de contraste inversé ou non inversé, préalablement acquise et mémorisée, sur l'image radioscopique avec possibilité de changer le pourcentage de mélange de l'image de référence.
- La radioscopie en mode dit "Road-Map" est une radioscopie soustraite. Une image plane soustraite est générée durant la radioscopie pour servir de masque aux radioscopies suivantes. Dans le domaine du vasculaire, une injection de produit de contraste pendant la génération de l'image soustraite durant la radioscopie réalise une cartographie vasculaire qui servira de masque de référence aux radioscopies suivantes. L'image opacifiée des vaisseaux est soustraite de l'image radioscopique active avec possibilité de mélanger en proportion variable un fond anatomique à l'image soustraite.

Les radioscopies en mode superposition et dit "Road-Map" réalisent une assistance au radio-guidage selon le plan de l'image de référence, c'est à dire le plan de projection de l'image déterminé par la position de l'arceau, la position de la région anatomique d'intérêt qui dépend de la position de la table d'examen, et selon l'agrandissement et l'échelle de l'image de référence qui dépendent de la valeur du champ de vue et de l'agrandissement géométrique déterminé par le rapport entre la distance focale de la source de rayons X aux moyens d'enregistrement, et, la distance de la source de rayons X à l'objet à radiographier. Ces deux modes de radioscopie présentent plusieurs inconvénients.

D'une part, à toute modification du plan de l'image de référence, de la position de la région anatomique d'intérêt, de l'agrandissement ou de l'échelle de l'image, l'opérateur doit procéder soit à l'acquisition et la mémorisation d'une nouvelle image de référence dans le cadre de la radioscopie en mode superposition, soit à la génération d'une nouvelle image soustraite de référence dans le cadre de la radioscopie en mode dit "Road-Map". Ces manipulations itératives ont pour conséquence d'allonger les temps d'intervention et d'irradiation, et d'augmenter les quantités de produit de contraste injectées chez le patient.

D'autre part, en cours d'intervention, lors de l'acquisition ou de la génération de nouvelles images de référence soustraites dans les cadres respectifs de la radioscopie en mode superposition avec image de référence soustraite et de la radioscopie en mode dit "Road-Map", il y a une perte d'information concernant la visualisation des instruments et du matériel de traitement radio-opaques en place par leur soustraction avec l'image de référence. Dans le cadre de la radioscopie en mode superposition avec image de référence non soustraite, la définition et la différenciation des structures anatomiques adjacentes dépendent de la différence de radio-opacité entre ces structures et posent problème lorsque la radio-opacité de celles-ci sont peu différentes ou pas assez différentes telle une structure vasculaire, canalaire ou cavitaire opacifiée par rapport à une structure osseuse adjacente.

Les radioscopies en mode superposition et dit "Road-Map" offrent une assistance au radioguidage basée sur des images de référence planes, figées dans le plan de référence, nécessitant d'être acquises ou générées à priori. Ces images de référence ne donnent pas d'information sur la troisième dimension de la région d'intérêt, ce qui représente un caractère limitatif et restrictif de l'assistance au radioguidage par ces deux modes de radioscopie.

Le document DE 10047314 A1 divulgue un procédé de navigation et un dispositif de radiographie destiné à obtenir un contraste virtuel pour l'angiographie augmentée. Les préambules des revendications 1 et 12 sont basés sur son contenu.

Un but de l'invention est de fournir un système de navigation amélioré par rapport à tout ou partie de cette problématique.

A cet effet, on prévoit, selon l'invention, un procédé de navigation tel que décrit dans la revendication 1.

Ainsi, dès que l'un des paramètres cités est modifié, le procédé recalcule en temps réel sans autre intervention les volumes et images de projection de volume affichés. L'utilisateur a donc toujours en temps réel une visualisation optimale, soit du volume et/ou de l'image de projection du volume de la région d'intérêt telle que la « voit » le dispositif de radiographie, et ce sans qu'aucun moyen de graphie ou scopie supplémentaire soit nécessaire, réduisant d'autant la dose de rayonnement émis en cours d'intervention, soit du volume résultant et/ou de l'image résultante de la superposition ou de la soustraction, respectivement, selon une coupe plane déterminée dans le volume et/ou sur l'image de projection de volume, de l'image de scopie de paramétrage correspondant, ce qui permet à l'utilisateur d'optimiser en temps réel le guidage de son instrumentation, le contrôle du déroulement de son geste technique et l'évaluation du résultat du geste technique.

Avantageusement mais facultativement, le procédé présente l'une au moins des caractéristiques supplémentaires suivantes :
- l'étape b) comporte des sous-étapes de :
   - b1) lecture dans des moyens de mémorisation du dispositif de radiographie d'une position (x,y,z) du support, d'une position (α,β,γ) de la source et des moyens d'enregistrement, du champ de vue (FOV), des distances focale (DF) et objet (DO); et,
   - b2) calcul de l'image de projection et/ou du sous-volume en fonction des paramètres ainsi lus.
- l'étape b) comporte des sous étapes de :
   - b1) lecture dans des moyens de mémorisation du dispositif de radiographie d'une position (x,y,z) du support, d'une position (α,β,γ) de la source et des moyens d'enregistrement ;
   - b2) calcul en fonction des ces positions d'un sous-volume V2 du volume V1 ;
   - b3) lecture dans les moyens de mémorisation du dispositif de radiographie du champ de vue (FOV), des distances focale (DF) et objet (DO) ;
   - b4) calcul en fonction du champ de vue (FOV), des distances focale (DF) et objet (DO) d'un volume corrigé V3 du sous-volume V2 ; et,
   - b5) calcul éventuel de l'image de projection à partir du volume corrigé V3.
- le volume corrigé V3 est calculé suivant un agrandissement géométrique et une mise à l'échelle en fonction du champ de vue (FOV) et des distances focale (DF) et objet (DO).
- lors de l'étape b2), une image de projection IP2 du sous volume V2 est, en outre, calculée en fonction desdites positions.
- lors de l'étape b5), l'image de projection IP3 est une image corrigée de l'image de projection IP2 en fonction du champ de vue (FOV), des distance focale (DF) et objet (DO).
- le calcul de la correction s'effectue par application d'une fonction géométrique d'agrandissement.
- le calcul du sous-volume V2 comporte des étapes de :
   i) détermination dans le volume V1 d'un axe d'incidence en fonction de la position (α,β,γ) de la source et des moyens d'enregistrement relativement à un référentiel du dispositif de radiographie dont une origine est un isocentre dudit dispositif de radiographie ;
   ii) détermination dans le volume V1 d'un centre du sous-volume V2 en fonction de la position (x,y,z) du support ; et,
   iii) calcul et reconstruction du sous-volume V2 à partir du volume V1 suivant un axe de reconstruction parallèle à l'axe d'incidence.
- le sous-volume V2 présente des dimensions n_{x'} par n_{y'} par n_{z'} définies par un opérateur.
- l'étape a) comporte des sous-étapes de :
   - a1) acquisition d'une pile de coupes de la région d'intérêt ; et,
   - a2) reconstruction du volume V1 sous la forme d'une matrice tridimensionnelle de voxels.
- l'étape c) comporte des sous-étapes de :
   c1) Lecture de l'image de scopie dans des moyens de mémorisation du dispositif de radiographie de l'image de scopie ;
   c2) Superposition ou soustraction à l'image de projection et/ou du sous-volume selon une coupe plane déterminée de l'image de scopie.

On prévoit aussi, selon l'invention, un dispositif de radiographie tel que décrit dans la revendication 12.

D'autres caractéristiques et avantages de l'invention apparaîtront lors de la description ci-après d'un mode de réalisation préféré. Aux dessins annexés :
- La figure 1 représente schématiquement le positionnement d'une région d'intérêt au sein d'un appareil de radiographie de type angiographie mettant en oeuvre le procédé de la divulgation,
- La figure 2 est un logigramme du procédé selon la divulgation,
- La figure 3 est un logigramme détaillé des différentes fonctions de la figure 2,
- Les figures 4a, 4b, 4c sont un exemple illustratif du résultat du procédé selon la divulgation,
- Les figures 5 et 6 sont des exemples illustratifs du résultat du calcul de l'image de projection en M.I.P. (Maximum Intensity Projection ou projection à maximum d'intensité) à partir du volume initial de la région d'intérêt selon le procédé de la divulgation sous différentes positions de l'appareil de radiographie par rapport à la région d'intérêt.

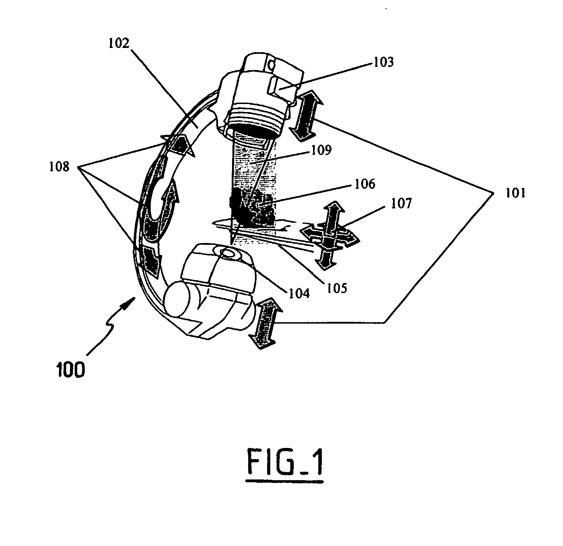

En référence à la figure 1, nous allons décrire un cadre d'application du procédé selon l'invention. Un appareil de radiographie 100 comprend un arceau 102 et un support 105, ici une table, apte à recevoir un objet 106, ici la tête d'un patient destinée à être radiographiée par l'appareil de radiographie 100 en vue d'une intervention au niveau d'une région anatomique d'intérêt, par exemple. L'arceau 102, en forme essentiellement de demi-cercle, comprend à l'une de ses extrémités une source de rayons X 104 et à son autre extrémité un capteur de rayons X 103 apte à faire l'acquisition de clichés radiographiques et d'images radioscopiques de la région d'intérêt préalablement positionnée dans le cône de rayons X 109 émis par la source 104. En fonctionnement, la surface utile du capteur 103 est en regard de la source de rayons X 104. La source de rayons X 104 et le capteur de rayons X 103 sont capables de se rapprocher ou de s'éloigner l'un de l'autre (schématisés par les flèches 101). Les positions relatives de la source de rayons X 104 et du capteur de rayons X 103 sont matérialisées par la distance qui les sépare et représentées par le paramètre distance focale (DF) que l'appareil d'angiographie 100 enregistre en permanence dans des moyens de mémorisation prévus à cet effet (non représentés). Ainsi que les positions relatives de la source de rayons X 104 et de la région d'intérêt de l'objet 106 à radiographier sont matérialisées par la distance qui les sépare et représentées par le paramètre distance objet (DO) que l'appareil d'angiographie 100 enregistre en permanence dans des moyens de mémorisation prévus à cet effet (non représentés).

Le champ de vue, dont les valeurs possibles sont prédéterminées en fonction de l'équipement radiologique 100, est défini par un paramètre (FOV) qu'enregistre en permanence l'appareil d'angiographie 100 dans des moyens de mémorisation prévus à cet effet (non représentés).

D'autre part, l'arceau 102 est capable de se déplacer selon les trois rotations de l'espace schématisées par les flèches 108. Cette position spatiale de l'arceau est représentée par les coordonnées angulaires (α, β, γ) qu'enregistre en permanence l'appareil de radiographie 100 dans des moyens de mémorisation prévus à cet effet (non représentés). La table support 105 est apte à se déplacer selon les trois translations de l'espace schématisées par l'ensemble de flèches 107. Comme précédemment, la position de la table support 105 est représentée par des coordonnées rectangulaires (x,y,z) que l'appareil de radiographie 100 enregistre en permanence dans des moyens de mémorisation prévus à cet effet (non représentés).

L'ensemble de ces paramètres, coordonnées rectangulaires (x,y,z) de la table 105, coordonnées angulaires (α, β, γ) de l'arceau 102, distance focale (DF), distance objet (DO) et champ de vue (FOV), que l'opérateur, en cours d'intervention, modifie de manière quasi-permanente, vont piloter le procédé selon l'invention que nous allons maintenant décrire.

### Définitions :

La position de l'origine O du référentiel de l'appareil de radiographie 100 est l'isocentre représenté par le point d'intersection des droites virtuelles passant par l'axe du tube radiogène formant la source de rayons X 104, et le centre de l'amplificateur de brillance comportant le capteur de rayons X 103 pour deux positions différentes de l'arceau 102.

Les coordonnées spatiales de l'arceau 102 sont déterminées par des coordonnées angulaires (α, β, γ). La position de l'origine du référentiel de l'arceau 102 de l'appareil de radiographie 100 est l'isocentre O. L'origine des coordonnées angulaires (a = 0°, p = 0°, y = 0°) est définie par la position verticale à 0 degré d'angle d'inclinaison latérale droite et gauche, et d'inclinaison longitudinale antérieure et postérieure (ou cranio-caudale ou caudo-craniale) de l'arceau 102 par rapport au support table 105 apte à recevoir l'objet à radiographier 106.

Les coordonnées spatiales de la table 105 sont déterminées par des coordonnées rectangulaires (x,y,z). La position de l'origine 0' du référentiel de la table 105 de l'appareil de radiographie 100 et l'origine des coordonnées rectangulaires (x = 0, y = 0, z = 0) sont déterminées par la position de la table 105 lorsque la région d'intérêt de l'objet 106 est positionnée dans l'isocentre O pour la réalisation de la série d'acquisition des images par angiographie rotationnelle décrite ultérieurement.

Le paramètre champ de vue (FOV) de l'appareil de radiographie 100, dépend des caractéristiques de l'équipement de l'appareil de radiographie 100 utilisé, et prend, de manière préférentielle, l'une des valeurs 33, 22, 17, et 13 cm. La valeur de référence du champ de vue est celle prise en compte pour la réalisation de la série d'acquisition des images par angiographie rotationnelle.

Le paramètre distance focale (DF) et le paramètre distance objet (DO) sont des caractéristiques de longueur sur l'axe du tube radiogène formant la source de rayons X 104 passant par le centre de l'amplificateur de brillance comportant le capteur de rayons X 103. Les valeurs de référence des distances focale (DF) et objet (DO) sont celles prise en compte pour la réalisation de la série d'acquisition des images par angiographie rotationnelle.

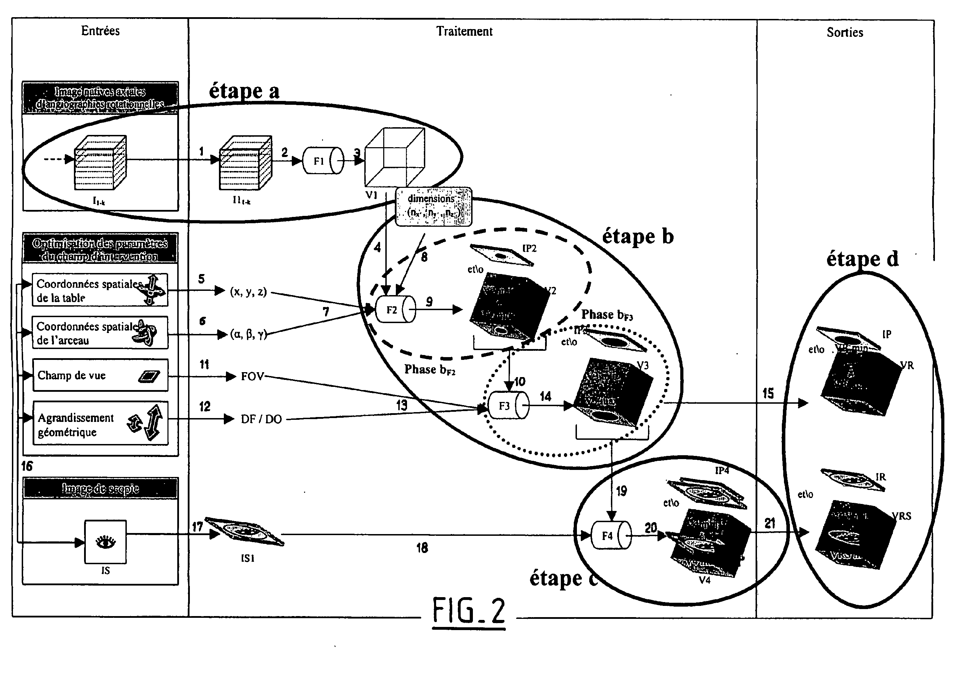

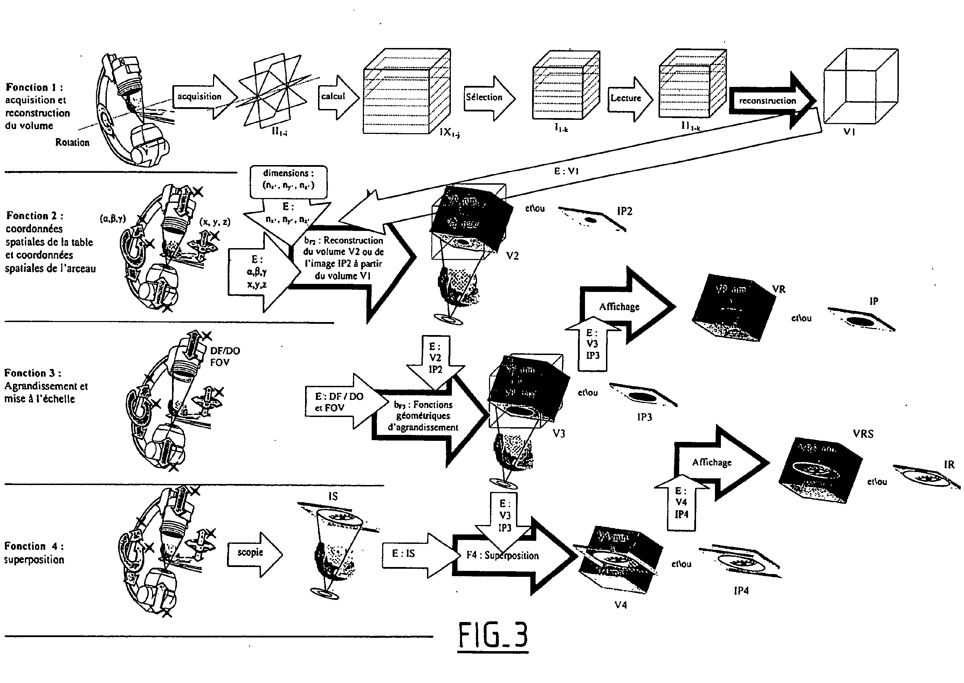

En référence aux figures 2 et 3, nous allons maintenant décrire le procédé selon la divulgation. Dans la figure 2, la colonne « entrées » du tableau indique l'ensemble des données fournies par l'appareil de radiographie 100 qui sont accessibles au procédé selon la divulgation. Le procédé selon la divulgation est schématisé dans la colonne « traitement » du tableau de la figure 2. La colonne « sorties » dudit tableau de la figure 2 représente les informations que le procédé fournit en retour à l'utilisateur.

La première étape du procédé selon l'invention correspond à l'étape a) qui se déroule préalablement à l'intervention elle-même et consiste en l'acquisition d'un certain nombre d'images de la région d'intérêt considérée et d'en reconstruire un volume tridimensionnel V1. Pour cela, le procédé utilise notamment la méthode de l'angiographie rotationnelle. Le principe d'une telle angiographie consiste en la réalisation d'une série d'images planes natives de projection de l'objet 106 contenant la région d'intérêt visualisée sous plusieurs incidences suivant la rotation de l'arceau, en vue d'être reconstruite en trois dimensions. La région d'intérêt devant faire l'objet d'une telle reconstruction est positionnée dans l'isocentre, comme illustré à la figure 1, puis l'objet 106 est exploré pendant une série d'acquisition d'images natives angulaires II₁₋ᵢ par rotation de l'arceau dans un plan de rotation déterminé afin d'être visualisée sous plusieurs incidences. Cela est illustré sur les deux premières images de la première ligne de la figure 3. Au cours de la réalisation de la série d'acquisition des images natives angulaires II₁₋ᵢ, les paramètres de l'appareil de radiographie 100 mis en jeu sont :
- différents paramètres prédéfinis avant le lancement de l'arceau que sont la fréquence d'acquisition des images (FREQ), le champ de vue (FOV), la distance focale (DF), la distance objet (DO) de la région d'intérêt de l'objet à radiographier 106 par rapport à la source de rayons X 104, l'étendue de la rotation de l'arceau 102 représentée par l'angle maximum de rotation (ANG-MAX), la vitesse de rotation de l'arceau 102 ainsi que les coordonnées rectangulaires (x,y,z) de la table support 105 de telle façon que la région d'intérêt de l'objet 106 à radiographier soit positionnée dans l'isocentre afin d'éviter qu'elle ne sorte du champ d'images visualisées lors de la rotation de l'arceau 102,
- des paramètres variables pendant la rotation de l'arceau 102 pour l'acquisition de la série d'images que sont les coordonnées angulaires (α,β,γ) de l'arceau 102 qui varient dans le plan de rotation.

Le nombre d'images obtenues par degré d'angle est déterminé par la vitesse de rotation de l'arceau 102 et la fréquence d'acquisition des images (FREQ). Le nombre i total d'images obtenues est déterminé par le nombre d'images par degré d'angles et l'étendue de la rotation de l'arceau 102 (ANG-MAX). Les images natives angulaires II₁₋ᵢ de projection d'incidences différentes de la région d'intérêt de l'objet 106 résultant de la série d'acquisition par angiographie rotationnelle sont visualisées perpendiculairement au plan de rotation de l'arceau 102, sous plusieurs incidences selon la position de l'arceau 102 au cours de la rotation permettant l'acquisition des images sous des angles de vue différents.

Ensuite, dans une sous-étape suivante, l'ensemble des images natives angulaires II₁₋ᵢ sont transformées en images natives axiales IX₁₋ᵢ. Les images natives angulaires II₁₋ᵢ, de projection d'incidences différentes de l'objet 106 contenant la région d'intérêt, obtenues par rotation de l'arceau 102 sont recalculées et reconstruites en projection axiale IX₁₋ᵢ afin d'obtenir un empilement d'images selon un axe prédéterminé en vue d'être reconstruites en trois dimensions en prenant en compte la totalité des images IX₁₋ᵢ ou une partie de ces images après sélection d'une pile d'images I₁₋ₖ (k étant compris entre 1 à j) correspondant à la région d'intérêt devant faire l'objet d'une intervention. Ces étapes sont effectuées directement par l'appareil de radiographie 100.

L'ensemble de ces images natives axiales I₁₋ₖ d'angiographie rotationnelle est récupéré par le procédé selon l'invention (flèche 1; figure 2) dans des moyens de mémorisation de l'appareil de radiographie 100 où elles sont stockées. Ensuite, ces images natives axiales servent de données d'entrée I1₁₋ₖ (flèche 2) à une fonction F1 de reconstruction. Cette fonction F1 permet la reconstruction en trois dimensions de manière à obtenir un volume de la région d'intérêt de l'objet 106 à partir de ces données d'entrée images natives axiales I1₁₋ₖ. Le volume V1 ainsi obtenu et correspondant aux données de sorties de la fonction F1 (flèche 3) est composé d'un ensemble de plusieurs voxels.

Le voxel est l'unité de volume correspondant au plus petit élément d'un espace tridimensionnel auquel on peut attribuer des caractéristiques individuelles, telles que la couleur ou l'intensité. Le terme voxel est l'acronyme anglais de *volume cell element (ou élément* cellule de *volume).* L'espace tridimensionnel est ainsi découpé en cubes élémentaires et chaque objet est décrit par les cubes qui le compose. Ainsi, le volume V1 est une matrice tridimensionnelle de 1 voxels par h voxels par p voxels. L'obtention de cette matrice tridimensionnelle représentant le volume V1 constitue l'aboutissement de l'étape a) du procédé selon l'invention.

Les étapes suivantes b), c) et d) du procédé selon l'invention s'effectuent préférentiellement en per-opératoire, alors que l'opérateur est en train d'intervenir sur le patient.

La deuxième étape du procédé selon l'invention correspond à l'étape b) et est constituée des phases b_{F2}) et b_{F3}) correspondant respectivement aux fonctions F2 et F3 du procédé que nous allons maintenant décrire.

Au cours de la phase b_{F2}), les données d'entrée utilisées par la fonction F2 sont d'une part la matrice tridimensionnelle du volume V1 (flèche 4) et d'autre part les coordonnées rectangulaires (x,y,z) (flèche 7), à l'instant t, de la table support 105, lues (flèche 5) dans les moyens de mémorisation de ces coordonnées rectangulaires de l'appareil de radiographie 100, illustrant la position de la table 105 à l'instant t, ainsi que les coordonnées angulaires (α, β, γ) (flèche 7), à l'instant t, de l'arceau 102, lues (flèche 6) dans les moyens de mémorisation de ces coordonnées angulaires de l'appareil de radiographie 100, illustrant la position de l'arceau 102 à l'instant t. Une dernière donnée d'entrée est éventuellement fournie à la fonction F2 (flèche 8) et correspond aux dimensions (n_{x'} n_{y'}, n_{z'}) d'un volume V2 calculé et reconstruit par la fonction F2 à partir du volume V1. Ces paramètres n_{x'}, n_{y'}, n_{z'} sont variables et déterminés, à l'instant t, par l'utilisateur lui-même. De préférence, ces paramètres n_{x'}, n_{y'}, n_{z'} sont exprimés en voxel et peuvent être compris entre 1 voxel et un nombre maximum de voxels permettant le calcul et la reconstruction du volume V2 à partir de tout le volume V1. Par la suite, le volume minimal V2min calculé correspond aux valeurs minimales de (n_{x'}, n_{y'}, n_{z'}) (c'est-à-dire 1 voxel) et le volume maximal V2max calculé correspond aux valeurs maximales possibles de (n_{x'}, n_{y'}, n_{z'}) permettant de reconstruire le volume V2 à partir du volume V1 en entier.

A partir de l'ensemble de ces données d'entrée, la fonction F2 calcule et reconstruit, à partir du volume V1 à l'instant t, le volume V2 ainsi qu'éventuellement, une image de projection IP2 du volume V2 correspondant aux coordonnées (x,y,z) de la table 105 et (α, β, γ) de l'arceau 102, et aux dimensions (n_{x'}, n_{y'}, n_{z'}) du volume V2. En sortie de la fonction F2, les données du volume V2 et de l'éventuelle image de projection IP2 du volume V2 sont disponibles (le volume V2 étant compris entre un volume V2min et un volume V2max correspondant aux valeurs extrêmes de n_{x'}, n_{y'}, n_{z'}) (flèche 9). Le volume V2 ainsi obtenu est donc un volume reconstruit à partir du volume VI et paramétré, à l'instant t, par les coordonnées (x,y,z) de la table support 105 et (α, β, γ) de l'arceau 102, ainsi que les dimensions (n_{x'}, n_{y'}, n_{z'}) allant de 1 voxel déterminant un volume V2min d'un voxel reconstruit à partir du volume V1, aux dimensions maximales déterminant un volume V2max reconstruit à partir de tout le volume V1.

Le calcul et la reconstruction du volume V2 à partir du volume V1 sont effectués, préférentiellement, selon l'algorithme suivant :
- détermination dans le volume V1 de l'axe d'incidence en fonction de (α, β, γ) relativement au référentiel de la salle d'angiographie 100 dont l'origine est l'isocentre, et, de la position du centre du volume V2 en fonction de (x, y, z) relativement au référentiel du support table 105 dont l'origine est déterminée par la position de la table lors de la série d'acquisition des images devant servir à la reconstruction du volume V1 de la région d'intérêt de l'objet 106, comme indiqué précédemment dans les définitions ;
- initialisation par l'opérateur de préférence ou détermination des dimensions n_{x'} n_{y'} n_{z'} en nombre de voxels du volume V2; et,
- calcul et reconstruction à partir du volume V1, du volume V2 par interpolation trilinéaire entre les voxels d'un ensemble de voxels à considérer du volume V1, dont le centre à été précédemment déterminé, de dimension (n_{x'}, n_{y'}, n_{z'}) voxels, suivant un axe de reconstruction représenté par l'axe d'incidence précédemment déterminé.

L'image de projection IP2 est calculée par projection selon l'axe d'incidence, sur un plan perpendiculaire à cet axe, du volume V2 ainsi déterminé.

Le volume V2 ainsi obtenu se présente sous la forme d'une matrice tridimensionnelle de n_{x'} voxels par n_{y'} voxels par n_{z'} voxels. Ce volume V2 et l'image de projection IP2 de ce volume V2 servent de données d'entrée pour une fonction F3 constituant principalement la phase b_{F3}) suivante de l'étape b) du procédé selon l'invention (flèche 10). Trois autres paramètres servent de données d'entrée (flèche 13) à cette fonction F3 :
- le paramètre (FOV) (flèche 13), à l'instant t, du champ de vue, lu (flèche 11) dans les moyens de mémorisation de ce paramètre de l'appareil de radiographie 100,
- le paramètre (DF) (flèche 13), à l'instant t, de la distance focale, lu (flèche 12) dans les moyens de mémorisation de ce paramètre de l'appareil de radiographie 100, et
- la paramètre (DO) (flèche 13), à l'instant t, de la distance objet, lu (flèche 12) dans des moyens de mémorisation de ce paramètre de l'appareil de radiographie 100.

La position de la région d'intérêt de l'objet 106 à radiographier par rapport à la source de rayons X 104 et au capteur de rayons X 103 à l'instant t détermine le paramètre agrandissement géométrique (DF/DO), à l'instant t, défini par le rapport entre la distance focale (DF) à l'instant t, et la distance objet (DO) à l'instant t.

A partir de ces différentes données d'entrée la fonction F3 calcule, à l'instant t, l'agrandissement géométrique et la mise à l'échelle du volume V2 reconstruit à partir du volume V1, ainsi que de l'image de projection IP2 du volume V2. Pour cela, selon les paramètres champ de vue(FOV), distances objet(DO) et focale (DF), la fonction F3 applique la fonction géométrique d'agrandissement, en l'occurrence la fonction géométrique de Thalès tenant compte que le rapport entre une dimension dans le volume V2 reconstruit à partir du volume V1 de la région d'intérêt de l'objet 106 radiographié ou une dimension sur l'image de projection IP2 du volume V2, et, la dimension à l'endroit correspondant dans la région d'intérêt, est égal au rapport entre la distance focale (DF) et la distance objet (DO) de la source de rayons X 104 à l'endroit correspondant dans la région d'intérêt de l'objet 106 où la dimension est prise en compte.

En sortie (flèche 14), la fonction F3 fournit un volume corrigé V3 du volume V2 et une image de projection IP3 du volume V3 ou une image de projection corrigée IP3 de l'image de projection IP2 du volume V2. Le volume V3 est, in fine, un volume calculé et reconstruit à partir du volume V1 et paramétré à l'instant t par les coordonnées (x, y, z) de la table 105, et (α, β, γ) de l'arceau 102, les paramètres d'agrandissement géométrique et de mise à l'échelle du champ de vue (FOV), de la distance objet (DO) et de la distance focale (DF), ainsi que les dimensions (n_{x'}, n_{y'}, n_{z'}) allant de 1 voxel déterminant un volume V3min d'un voxel reconstruit à partir du volume V1, aux dimensions maximales déterminant un volume V3max reconstruit à partir de tout le volume V1. Comme pour les volumes V1 et V2 précédents, le volume V3 calculé se présente sous la forme d'une matrice tridimensionnelle de voxels.

Une fois le volume V3 et l'image de projection IP3 du volume V3 calculés, le procédé selon l'invention peut transférer le volume V3 et/ou l'image de projection IP3 pour affichage (flèche 15) sur des moyens d'affichage aptes à être consultés à l'instant t par l'utilisateur. Ceci permet à l'utilisateur de voir à l'instant t, sur des moyens d'affichage, un volume VR de la région d'intérêt (volume V3 transmis) et/ou l'image de projection IP (image IP3 transmise) du volume de la région d'intérêt, correspondant à la position relative du support 105, de l'arceau 102, et aux valeurs des paramètres champ de vue (FOV), distance objet (DO), distance focale (DF) et dimensions (n_{x'}, n_{y'}, n_{z'}) à cet instant t. Il est à noter qu'aucun moyen de graphie ou de scopie n'a été mis en oeuvre pour délivrer la représentation de ce volume et/ de l'image de projection de ce volume.

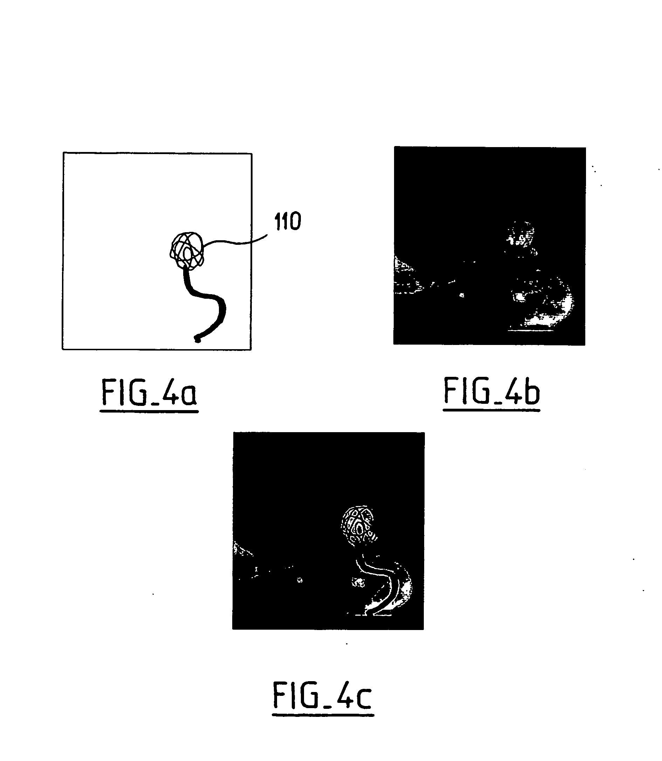

Au cours de l'intervention, l'opérateur utilisateur du procédé selon l'invention peut introduire dans la région d'intérêt un ou plusieurs instruments 110 (Figure 4a) dont il souhaite, à l'instant t, connaître la position exacte. Pour cela, l'opérateur utilise l'appareil de radiographie pour saisir une image de scopie (IS) (flèche 16) à l'instant t, alors que l'arceau 102 se trouve aux coordonnées angulaires (α, β, γ), la table de support 105 aux coordonnées rectangulaires (x,y,z), et le capteur 103 et la source de rayon X 104 positionnés de manière à avoir le champ de vue (FOV), la distance objet (DO) et la distance focale (DF). L'image de scopie IS1 ainsi saisie est lue (flèche 17), à cet instant t, dans des moyens de mémorisation des données de l'image de scopie de l'appareil de radiographie 100, accessibles au procédé selon l'invention. Les données correspondantes à cette image de scopie IS1 servent de données d'entrée(flèche 18) lors de l'étape c) du procédé selon l'invention à une fonction F4. Cette fonction F4 comprend ainsi, comme données d'entrée, le volume V3 et/ou l'image de projection IP3 du volume V3 précédemment calculés (flèche 19) et l'image de scopie IS1, saisie et lue à l'instant t dans les moyens de mémorisation des données de l'image de scopie de l'appareil de radiographie 100. La fonction F4 effectue la superposition ou la soustraction, à l'instant t, dans le volume V3 selon une coupe plane déterminée et/ou sur l'image de projection IP3 du volume V3 précédemment calculés, de l'image de scopie IS1 de paramétrages correspondants (flèche 16) relativement aux coordonnées (x,y,z) de la table 105 et (α, β, γ) de l'arceau 102 ainsi que des valeurs du champ de vue (FOV), de la distance objet (DO) et de la distance focale (DF). Pour cela, à l'instant t, la fonction F4 superpose ou soustrait dans le volume V3 selon une coupe plane déterminée et/ou sur l'image de projection IP3 du volume V3, l'image de scopie IS1, et/ou calcule une image de projection IP4 d'un volume V4 résultant de la superposition ou de la soustraction dans le volume V3 selon une coupe plane déterminée de l'image de scopie IS1, la projection s'effectuant dans un plan parallèle au plan de l'image de scopie IS1 et selon une direction perpendiculaire à ladite image de scopie IS1. La fonction F4 fournit en sortie (flèche 20) le volume V4 et/ou l'image de projection IP4 résultant de la superposition ou soustraction précédemment décrite. Le procédé selon l'invention peut transférer le volume V4 (soit un volume VRS) et/ou l'image de projection IP4 (soit une image IR) de manière à les afficher (flèche 21) sur des moyens d'affichage aptes à être consultés, à l'instant t, par l'utilisateur. Ceci permet à l'utilisateur de voir à l'instant t, sur des moyens d'affichage, le volume VRS de la région d'intérêt et/ou l'image de projection IR du volume de la région d'intérêt correspondant à la position relative de la support 105, de l'arceau 102, et aux valeurs des paramètres champ de vue (FOV), distance objet (DO), distance focale (DF), et dimensions (n_{x'}, n_{y'}, n_{z'}) à cet instant t. Ainsi, l'utilisateur connaît la position exacte selon les paramètres prédéterminés à cet instant t, des instruments 110 au sein de la région d'intérêt considérée, comme cela est illustré en figures 4a à 4c.

En figure 4a, est illustrée une image de scopie IS1 prise à un instant t, visualisant les instruments et matériels 110. La figure 4b présentent une image de projection IP3 d'une structure artérielle comportant un anévrysme intracrânien, calculé comme précédemment décrit, correspondants aux paramètres (x, y, z), (α, β, γ), (FOV), (DO), (DF) et (n_{x'}, n_{y'}, n_{z'}) associés à l'image scopie IS1 de la figure 4a. En figure 4c, est illustré une image de projection IRS résultante de la superposition effectuée par la fonction F4 lors de l'étape c) du procédé selon l'invention où l'image de scopie IS1 de la figure 4a a été superposée à l'image de projection IP3 de la figure 4b illustrant ainsi la façon dont l'opérateur vérifie et contrôle le positionnement de son instrumentation 110 lors d'une intervention sur un anévrysme comme illustré.

Ensuite à l'instant t+δt, l'opérateur :
- soit déplace ses instruments 110 et souhaite suivre leur déplacement par la saisie, à l'instant t+δt, d'une nouvelle image de scopie, ce qui a pour conséquence de faire effectuer de nouveau, à l'instant t+δt, au procédé selon l'invention, l'étape c) précédemment décrite, et donc de mettre à jour, à l'instant t+δt, le volume VRS et/ou l'image de projection IR affichés, par les moyens d'affichage accessibles à l'utilisateur ;
- et/ou modifie la position relative de l'arceau 102 et/ou de la table 105, ce qui a pour conséquence de faire effectuer de nouveau, à l'instant t+δt, au procédé selon l'invention, la phase bF₂ de l'étape b) précédemment décrite, et donc de mettre à jour, à l'instant t+δt, le volume VR et/ou l'image de projection IP affichés, par les moyens d'affichage accessibles à l'utilisateur. Une nouvelle saisie éventuelle d'une image de scopie met en jeu l'étape c) ;
- et/ou modifie la distance focale (DF) et/ou la distance objet (DO,) ce qui a pour conséquence de faire effectuer de nouveau, à l'instant t+δt, au procédé selon l'invention, la phase bF₃ de l'étape b) précédemment décrite, et donc de mettre à jour, à l'instant t+δt, le volume VR et/ou l'image de projection IP affichés, par les moyens d'affichage accessibles à l'utilisateur. Une nouvelle saisie éventuelle d'une image de scopie met en jeu l'étape c).

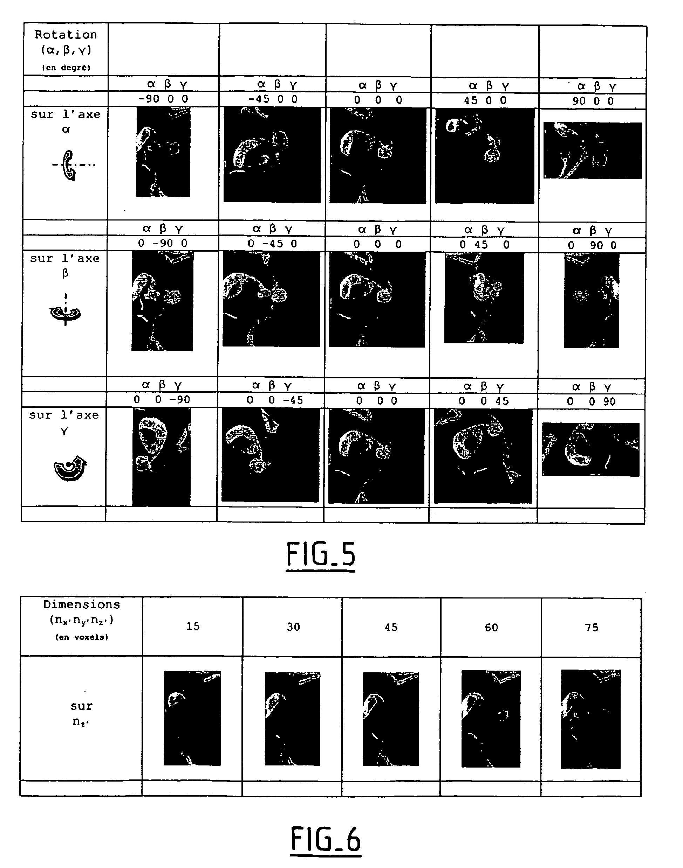

Dans l'exemple donné, les figures 5 et 6 représentent le résultat du calcul d'une image de projection IP en fonction de différentes positions de l'arceau 102. La première ligne d'images de la figure 5 correspond à une variation de l'angle α de l'arceau 102 respectivement à -90°, -45°, 0°, 45° et 90° alors que les autres angles β, γ restent inchangés à 0°. La seconde ligne d'images illustre une variation similaire de l'angle β alors que α, γ sont fixés à 0°. De même, pour la troisième ligne d'images, α, β sont fixés à 0° et γ varie. Pour l'ensemble de ces images, le volume initial V1 a une taille de 1=256 voxels par h=256 voxels par p=153 voxels.

La figure 6 illustre pour des coordonnées spatiales fixes (α, β, γ) et (x, y, z) le calcul d'une image de projection IP suivant différentes valeurs pour n_{z'} (n_{x'} et n_{y'} étant inchangés), respectivement 15 voxels, 30 voxels, 45 voxels, 60 voxels et 75 voxels.

D'une manière pratique et préférentielle, afin de valider le procédé décrit précédemment, le langage de programmation utilisé est le langage Java. Il est conçu par l'association de plusieurs modules logiciels ou plugiciels (ou encore plugins selon le terme anglo-saxon consacré) ajoutant chacun des fonctionnalités telles que nous les avons décrites précédemment.

De manière préférentielle, ils permettent des fonctions de base pour le traitement d'images de tout format dont en particulier le format DICOM utilisé en radiologie. Ces fonctions sont la lecture, l'affichage, l'édition, l'analyse, le traitement, la sauvegarde et l'impression de telles images. Ils permettent de calculer des statistiques sur un pixel, ou un voxel, ou sur une aire définie par sélection. Des mesures de distance et d'angle peuvent être effectuées. Ils peuvent effectuer aussi des traitements sur les densités et supportent l'essentiel des fonctions standards d'imagerie comme la modification du contraste, la détection des bords ou des filtres médians. Ils peuvent en outre effectuer des transformations géométriques telles que l'agrandissement, le changement d'échelle, la rotation ; toutes les fonctions d'analyse et de traitement précédentes étant utilisables à n'importe quel agrandissement.

De plus, chaque fonction spécifique au procédé selon l'invention est réalisée par un plugiciel dédié. Préférentiellement, un plugiciel permet le calcul et la reconstruction de coupes orthogonales par rapport à un axe donné d'un volume ou d'une région d'intérêt.

Un autre plugiciel s'occupe du calcul et de la reconstruction d'un volume, et de son image de projection associée, en effectuant un rendu volumique et en travaillant ledit rendu sur tout ensemble de voxels et/ou sur toute pile de coupes. Ce plugiciel permet la reconstruction de volume selon un axe déterminé. Le volume peut être tourné, agrandi, ou réduit. Pour cela, l'interpolation du volume effectuant le rendu est une interpolation trilinéaire, connue en soi, excepté pour les coupes d'extrémité de la pile et/ou les voxels d'extrémité de l'ensemble de voxels où cette interpolation trilinaire est impossible. Dans ce cas, une interpolation du plus proche voisin, connue en soi, est utilisée en remplacement de l'interpolation trilinéaire.

Un autre plugiciel permet d'effectuer une projection selon un axe, à titre d'exemple en maximum d'intensité projection (M.I.P). Cela permet de calculer l'image de projection IP3 du volume V3, par exemple.

Le procédé selon l'invention précédemment décrit utilise les différents plugiciels précédemment cités de manière à calculer l'image de projection d'un volume. Pour cela à chaque changement de valeur des paramètres (x, y, z) de position de la table support 105, ou de la position (α, β, γ) de l'arceau 102, ou encore du champ de vue (FOV), de la distance de l'objet (DO) par rapport à la source, de la distance focale (DF) ou des dimensions (n_{x'}, n_{y'}, n_{z'}) du volume recherché, (n_{x'}, n_{y'}, n_{z'}) déterminés par l'opérateur, le procédé selon l'invention utilise le plugiciel de reconstruction du volume, recalculé alors suivant la projection angulaire (α, β, γ) de la région d'intérêt, puis calcule l'agrandissement et la mise à l'échelle en fonction du champ de vue (FOV) et du rapport de la distance focale (DF) sur la distance de l'objet (DO) par rapport à la source, puis, à l'aide du plugiciel de projection, calcule l'image de la projection du volume, et affiche l'image de projection IP de ce volume sur des moyens d'affichages après ou non superposition ou soustraction de l'image de scopie IS1 associée.

L'imagerie tridimensionnelle acquise par angiographie rotationnelle, permet une meilleure compréhension de l'anatomie réelle d'une lésion, d'un organe, ou d'une partie d'un organe en permettant la visualisation de cette structure sous tous les angles de vue requis. Son intérêt est essentiellement diagnostique. Sur le plan thérapeutique, son intérêt est limité à l'optimisation des angles de vue pertinents, soit avant traitement pour définir à priori une stratégie thérapeutique, soit après traitement pour évaluer le résultat thérapeutique. L'utilisation de l'imagerie tridimensionnelle de référence en per-thérapeutique est un concept nouveau, jamais élaboré jusqu'alors en imagerie de projection, pour, à tout instant en cours d'intervention, adapter et ajuster ses décisions et stratégies thérapeutiques, assister et contrôler son geste technique et évaluer les résultats thérapeutiques.

Un cadre d'application du procédé selon l'invention est décrit selon une technique d'imagerie de projection à partir d'un appareil d'angiographie, pour l'investigation et le traitement endovasculaire d'un anévrysme intracrânien. La région d'intérêt est représentée par le réseau vasculaire artériel intracrânien afférent et porteur d'un anévrysme intracrânien.

Dans le domaine médical, les images permettant la reconstruction du volume de la région d'intérêt en trois dimensions peuvent être acquises par les techniques d'imagerie, y compris les méthodes de reconstruction endovirtuelle, sans être exhaustif:
1) les techniques d'imagerie de projection telle l'angiographie rotationnelle précédemment décrite,
2) les techniques d'imagerie en coupe telle la tomodensitométrie informatisée ou scanner, l'imagerie par résonance magnétique ou l'imagerie par ultra-son,
3) les techniques d'imagerie vidéo,
4) les techniques d'imagerie numérique de synthèse.

Les images permettant la reconstruction du volume de la région d'intérêt en trois dimensions peuvent provenir d'images ou de volumes préalablement retravaillés, sans être exhaustif, les reconstructions des images ou volumes à partir de toutes les techniques précédemment citées.

Les images actives temps réel peuvent être des images, sans être exhaustif :
1) radioscopiques des techniques radiologiques et angiographiques,
2) cinéscopiques des techniques d'imagerie de tomodensitométrie informatisées ou scanographique, par résonance magnétique, ou par ultrason,
3) videoscopiques des techniques d'imagerie par caméra vidéo telle l'endoscopique ou la coelioscopie,
4) tridimensionelles telles que la stéréoscopie ou la scopie-3D,
5) numériques pour caméra numérique ou images numériques de synthèse.

La technique d'imagerie produisant les images actives temps réel et celle permettant l'acquisition pour la reconstruction du volume de la région d'intérêt en trois dimensions peuvent être sans être exhaustif soit la même technique soit des techniques différentes et requièrant alors le repérage du volume de la région d'intérêt selon un référentiel soit interne soit externe au sujet dont dépend la région d'intérêt.

Les moyens d'affichages peuvent être, sans être exhaustif :
1) les affichages bidimensionnels sur lesquels des images de projections de volumes sont utilisées.
2) les affichages simulés tridimensionnels retransmettant une impression de volume.
3) les affichages tridimensionnels (de nouvelles technologies, notamment les systèmes holographiques) sur lesquels plusieurs volumes peuvent être confondus, ajoutés ou soustrait.

Lors de procédures de radiologie interventionnelle assistées par le procédé selon l'invention, la connaissance en temps réel des données sur la troisième dimension de la région anatomique d'intérêt dans sa globalité (volume et image de projection du volume de la région d'intérêt) ou en son sein en faisant apparaître une zone masquée de la région anatomique d'intérêt (volume et image de projection du volume d'une partie de la région d'intérêt), en temps réel (c'est à dire en per-procédure avec un temps de réponse rapide quasi-immédiat), d'une manière dynamique (c'est à dire en mouvement en rapport avec tout changement de paramétrage entrant dans la chaîne d'acquisition des images tel que la position de la table, de l'arceau et des valeurs du champ de vue, de la distance focale de la source de rayons X aux moyens d'enregistrement, ou de la distance de la région d'intérêt à radiographier par rapport à la source de rayons X pour un appareil radiologique de type angiographie), d'une manière interactive (c'est à dire adaptée à la demande de l'opérateur) et sous tous les angles de vue (c'est à dire en rapport avec toutes les incidences possibles de graphie ou de scopie pour un appareil radiologique de type angiographie, par exemple), qui, superposées ou soustraites aux données d'images des instruments et matériels radio-opaques obtenues par l'image de scopie active soustraite ou non soustraite, permet une optimisation des informations de la région d'intérêt et de la position des instruments et matériels radio-opaques au sein de la région d'intérêt et donc de prendre des décisions adaptées en temps réel et au fur et à mesure du déroulement de l'investigation ou de l'intervention concernant la détermination des champs de vue pertinents d'investigation ou d'intervention de la région d'intérêt, les stratégies d'investigation ou d'intervention, le guidage de l'instrumentation, le contrôle du déroulement du geste technique, et l'évaluation du résultat du geste technique. Les conséquences sont, d'une part, l'optimisation de la sécurisation et de l'efficacité de l'investigation ou de l'intervention, et, d'autre part, une diminution du temps de procédure opératoire, des quantités de produit de contraste iodé injectées chez le patient et de l'irradiation chez le patient et l'opérateur.

Bien entendu, on pourra apporter à l'invention de nombreuses modifications sans pour autant sortir du cadre de celle-ci.

## Revendications

1. Procédé de navigation au sein d'une région d'intérêt destiné à être mis en oeuvre dans un dispositif de radiographie (100) du Lype comportant une source (104) de rayons X, des moyens d'enregistrement (103) disposés en regard de la dite source et un support (105) sur lequel un objet (106) à radiographie comprenant la région d'intérêt est destiné à être positionné, le procédé comportant des étapes de :
a) acquisition de données tridimensionnelles d'images d'un volume (V1) de la région d'intérêt ;
b) calcul, à un instant t, d'une image bidimensionnelle de projection (I.P, I.P.2, IP3) de tout ou partie du volume (V1) et/ou d'une sous-volume (V2, V3, VR) dudit volume (V1) en fonction de paramètres comprenant les positions du support (105), de la source (104) et des moyens d'enregistrement (103), le champ de vue (FOV), les distances focale (DF) et objet (DO) ;
c) superposition ou soustraction de l'image de projection (IP, IP3) et/ou du sous-volume (V3, VR) selon une coupe plane déterminée à une image de scopie (IS1) associée audits paramètres à l'instant t ;
d) affichage sur des moyens d'affichage d'une image (TR) et/ou d'un volume (VRS) résultant de l'étape c), et/ou de l'image de projection (IP, IP2, IP3) et/ou du sous-volume (V2, V3, VR), **caractérisé en ce que** le procédé comporte la
e) répétition des étapes b), c) et d) à chaque changement de valeur dans lesdits paramètres.

2. Procédé selon la revendication 1, **caractérisé en ce que** l'étape b) comporte des sous-étapes de :
- b1) lecture dans des moyens de mémorisation du dispositif de radiographie d'une position (x, y, z) du support, d'une position (α, β, γ) de la source et des moyens d'enregistrement, du champ de vue (FOV), des distances focale (DF) et objet (DO) ; et,
- b2) calcul de l'image de projection, (IP, IP3) et/ou du sous-volume (V3, VR) en fonction des paramètres ainsi lus.

3. Procédé selon la revendication 1 ou 2, **caractérisé en ce que**, l'étape b) comporte des sous étapes de :
- b1) lecture dans des moyens de mémorisation du dispositif de radiographie d'une position (x, y, z) du support, d'une position (α, β, γ) de la source et des moyens d'enregistrement ;
- b2) calcul en fonction des ces positions d'un sous-volume V2 du volume V1 ;
- b3) lecture dans les moyens de mémorisation du dispositif de radiographie du champ de vue (FOV), des distances focale (DF) et objet (DO) ;
- b4) calcul en fonction du champ de vue (FOV), des distances focale (DF) et objet (DO) d'un volume corrigé V3 du sous-volume V2 ; et,
- b5) calcul éventuel de l'image de projection (IP, IP3) à partir du volume corrigé V3.

4. Procédé selon la revendication 3, **caractérisé en ce que** le volume corrigée V3 est calculé suivant un agrandissement géométrique et une mise à l'échelle en fonction du champ de vue (FOV) et des distances focale (DF) et objet (DO).

5. Procédé selon la revendication 3, **caractérisé en ce que**, lors de l'étape b2), une image de projection (IP2) du sous volume V2 est, en outre, calculée en fonction desdites positions.

6. Procédé selon la revendication 5, **caractérisé en ce que**, lors de l'étape b5), l'image de projection (IP, IP3) est une image corrigée de l'image de projection (IP2) en fonction du champ de vue (FOV), des distance focale (DF) et objet (DO).

7. Procédé selon la revendication 4 ou 6, **caractérisé en ce que** le calcul de la correction s'effectue par application d'une fonction géométrique d'agrandissement.

8. Procédé selon l'une des revendications 3 à 7, **caractérisé en ce que** le calcul du sous-volume V2 comporte des étapes de :
i) détermination dans le volume V1 d'un axe d'incidence en fonction de la position (α, β, γ) de la source (104) et des moyens d'enregistrement (103) relativement à un référentiel du dispositif de radiographie dont une origine est un isocentre dudit dispositif de radiographie ;
ii) détermination dans le volume V1 d'un centre du sous-volume V2 en fonction de la position (x, y, z ) du support (105) ; et,
iii) calcul et reconstruction du sous-volume V2 à partir du volume V1 suivant un axe de reconstruction parallèle à l'axe d'incidence.

9. Procédé selon l'une des revendications 3 à 8, **caractérisé en ce que** le sous-volume V2 présente des dimensions n_{x'} par n_{y'} par n_{z'} définies par un opérateur.

10. Procédé selon l'une des revendications précédentes, **caractérisé en ce que** l'étape a) comporte des sous-étapes de :
- a1) acquisition d'une pile de coupes de la région d'intérêt ; et ,
- a2) reconstruction du volume V1 sous la forme d'une matrice tridimensionnelle de voxels.

11. Procédé selon l'une des revendications 1 à 10, **caractérisé en ce que** l'étape c) comporte des sous-étapes de :
c1) Lecture de l'image de scopie IS1 dans des moyens de mémorisation du dispositif de radiographie de l'image de scopie IS1 ;
c2) Superposition ou soustraction à l'image de protection (IP, IP3) et/ou du sous-volume selon une coupe plane déterminée de l'image de scopie IS1.

12. Dispositif de radiographie du type comportant une source de rayons X, des moyens d'enregistrement disposés en regard de la dite source et un support sur lequel un objet à radiographier comprenant la région d'intérêt est destiné à être positionné, des moyens d'acquisition de données tridimensionnelles reliés aux moyens d'enregistrements, des moyens de calcul et des moyens d'affichage, **caractérisé en ce que** l'ensemble de ces moyens est agencés de sorte à mettre en oeuvre le procédé selon l'une des revendications précédentes.

## Claims

1. Method for navigation inside a region of interest, for use in a radiography unit (100) including an X-ray source (104), recording means (103) facing the source, and a support (105) on which an object (106) to be radiographed, containing the region of interest, can be positioned the method comprising the following steps:
a) acquiring three-dimensional image data of a volume (V1) of the region of interest;
b) calculating, at a time t, a two-dimensional projection image (IP, IP2, IP3) of all or part of the volume (V1) and/or a sub-volume (V2, V3, VR) of said volume (V1) according to the position of the support (105), the position of the source (104) and recording means (103), a field of vision (FOV), a focal distance (DF) and an object distance (DO);
c) superposing on, or subtracting from to the projection image (IP, IP3) and/or the sub-volume (V3, VR), according to a given plane section, a radioscopic image (1S1) associated with said parameters at the time t,
d) displaying on display means an image (IR) and/or a volume (VRS) resulting from step c), and/or the projection image (IP, IP2, IP3) and/or the sub-volume (V2, V3, VR), **characterised in that** the method includes
e) repeating steps b), c) and d) for each change of value in said parameters.

2. Method according to claim 1, **characterised in that** step b) includes the following sub-steps:
- b1) reading in the storage means of the radiography device a support position (x, y, z), a source and recording means position (α, β, γ) and the values of the field of vision (FOV), focal distance (DF) arid object distance (DO); and,
- b2) calculating the projection image (IP, IP3) and/or sub-volume (V3, VR) according to the read parameters.

3. Method according to any one of claims 1 and 2, **characterised in that** step b) includes the following sub-steps:
- b1) reading in the storage means of the radiography device a support position (x, y, z) and a source and recording means position (α, β, γ) ;
- b2) calculating a sub-volume V2 of volume V1, according to these positions;
- b3) reading in the storage means of the radiography device the values of field of vision (FOV), focal distance (DF) and object distance DO);
- b4) calculating a corrected volume V3 of sub-volume V2 according to the field of vision (FOV), the focal distance (DF) and the object distance (DO); and,
- b5) optionally calculating the projected image (IP, IP3) on the basis of the corrected volume V3.

4. Method according to claim 3, **characterised in that** the corrected volume V3 is calculated as a geometric enlargement and a scaling according to the field of vision (FOV), the focal distance (DF) and the object distance (DO).

5. Method according to claim 3, **characterised in that**, during step b2), a projection image (IP2) of sub-volume V2 is also calculated according to said positions.

6. Method according to claim 5, **characterised in that**, during step b5), the projection image (IP, IP3) is generated by correcting the protection image (IP2) according to the field of vision (FOV), the focal distance (DE) and the object distance (DO).

7. Method according to claim 4 or 6, **characterised in that** the calculation of correction is performed by use of a geometrical enlargement function.

8. A method according to any one of claims from 3 to 7, **characterised in that** the calculation of sub-volume V2 includes the following steps:
i) determining, in volume V1, an incidence axis depending on the position (α, β, γ) of the source (104) and of the recording means (103) relative to a reference system of the radiography device, an origin of which is an isocentre of said radiography device;
ii) determining in volume VI a centre of sub-volume V2 depending on the position (x, y, z) of support (105); and,
iii) calculating and reconstructing sub-volume V2 from volume V1 according to a reconstruction axis parallel to the incidence axis.

9. Method according to any one of claims 3 to 8, **characterised in that** the sub-volume V2 has dimensions nₓ, x n_{y}, x n_{z}, which are defined by an operator.

10. Method according to any one of the preceding claims, **characterised in that** step a) includes the following sub-steps:
- a1) acquiring of a set of sections through the region of interest; and,
- a2) reconstructing volume V1 in the form of a three-dimensional voxel matrix.

11. Method according to any one of claims 1 to 10, **characterised in that** step c) includes the following sub-steps:
c1) reading the radioscopic image IS1 in the storage means of the radiography device, and
c2) superposing said image on or subtracting said image from the projection image (IP, 123) and/or sub-volume along a given plane section of the radioscopic image IS1.

12. Radiography device, comprising an X-ray source, recording means facing said source, a support on which an object to be radiographed, containing a region of interest, can be positioned, three-dimensional data acquisition means connected to the recording means, computing means and display means, **characterised in that** said means are arranged together so as to implement the method according to any one of the preceding claims.

## Patentansprüche

1. Navigationsverfahren innerhalb einer Region von Bedeutung, das dazu bestimmt ist, in einem Radiographie-System (100) angewandt zu werden, das in der Art gestaltet ist, dass es eine Röntgenstrahlenquelle (104) und Speichervorrichtungen (103) umfasst, die gegenüber der besagten Quelle angeordnet sind, sowie einen Support (105), auf dem ein zu röntgender Gegenstand (106) umfassend die Region von Bedeutung angeordnet werden soll, wobei das Verfahren die folgenden Schritte umfasst:
a) die Erfassung dreidimensionaler Bilddaten eines Volumens (V1) der Region von Bedeutung;
b) die Berechnung, zu einem Zeitpunkt t, eines zweidimensionalen Projektionsbildes (IP, IP2, IP3) des gesamten Volumens (V1) oder eines Teils davon und/ oder eines Unter-Volumens (V2, V3, VR) des besagten Volumens (V1) in Anhängigkeit von den Parametern umfassend die Positionen des Supports (105), der Quelle (104) und der Speichervorrichtungen (103), das Sichtfeld (FOV), die Fokal- (DF) und Gegenstandsabstände (DO);
c) die Überlagerung oder die Subtraktion des Projektionsbildes (IP, IP3) und/ oder des unter-Volumens (V3, VR) in einem bestimmten ebenen Schnitt von einem Röntgenaufnahmebild (IS1), das den besagten Parametern zum Zeitpunkt t zugeordnet wird;
d) die Anzeige eines Bildes (TR) und/ oder eines Volumens (VRS) resultierend aus dem Schritt c), und/ oder des Projektionsbildes (IP, IP2, IP3) und/ oder des Unter-Volumens (V2, V3, VR) auf Anzeigevorrichtungen, **dadurch gekennzeichnet, dass** das Verfahren die
e) Wiederholung der Schritte b), c) und d) bei jeder Wertänderung in den besagten Parametern umfasst.

2. Verfahren nach Anspruch 1, **dadurch gekennzeichnet, dass** der Schritt b) die folgenden Unterschritte umfasst:
- b1) das Ablesen in den Speichervorrichtungen des Radiographie-Systems einer Postition (x, y, z) des Supports, einer Position (α, β, γ) der Quelle und der Speichervorrichtungen, des Sichtfeldes (FOV), der Fokal- (DF) und Gegenstandsabstände (DO); und,
- b2) die Berechnung des Projektionsbildes (IP, IP3) und/ oder des Unter-Volumens (V3, VR) in Abhängigkeit von den abgelesene Parametern.

3. Verfahren nach Anspruch 1 oder 2, **dadurch gekennzeichnet, dass** der Schritt b) die folgenden Unterschritte umfasst:
- b1) das Ablesen in den Speichervorrichtungen des Radiographie-Systems einer Position (x, y, z) des Supports, einer Position (α, β, γ) der Quelle und der Speichervorrichtungen;
- b2) die Berechnung eines Unter-Volumens V2 des Volumens V1 in Abhängigkeit von diesen Positionen;
- b3) das Ablesen in den Speichervorrichtungen des Radiographie-Systems des Sichtfeldes (FOV), der Fokal- (DF) und Gegenstandsabstände (DO);
- b4) die Berechnung eines korrigierten Volumens V3 des Unter-Volumens V2 in Abhängigkeit vom Sichtfeld (FOV), der Fokal- (DF) und Gegenstandsabstände (DO);
- b5) die eventuelle Berechnung des Projektionsbildes (IP, IP3) ausgehend vom korrigierten Volumen.

4. Verfahren nach Anspruch 3, **dadurch gekennzeichnet, dass** das korrigierte Volumen V3 gemäß einer geometrischen Vergrößerung und einer Normierung in Abhängigkeit vom Sichtfeld (FOV) und den Fokal- (DF) und Gegenstandsabständen (DO) berechnet wird.

5. Verfahren nach Anspruch 3, **dadurch gekennzeichnet, dass** beim Schritt b2) darüber hinaus ein Projektionsbild (IP2) des Unter-Volumens V2 in Abhängigkeit von den besagten Positionen berechnet wird.

6. Verfahren nach Anspruch 5, **dadurch gekennzeichnet, dass** das Projektionsbild (IP, IP3) beim Schritt b5) ein korrigiertes Bild des Projektionsbildes (IP2) in Abhängigkeit vom Sichtfeld (FOV) und den Fokal- (DF) und Gegenstandsabständen (DO) ist.

7. Verfahren nach Anspruch 4 oder 6, **dadurch gekennzeichnet, dass** die Berechnung der Korrektur durch die Abwendung einer geometrischen Vergrößerungsfunktion erfolgt.

8. Verfahren nach einem der Ansprüche 3 bis 7, **dadurch gekennzeichnet, dass** die Berechnung des Unter-Volumens V2 die folgenden Schritte umfasst:
i) die Bestimmung im Volumen V1 einer Lichteinfallachse in Abhängigkeit von der Position (α, β, γ) der Quelle (104) und der Speichervorrichtungen (103) in Bezug auf ein Bezugssystem des Radiographie-Systems, wobei einer der Ausgangspunkte ein Isozentrum des besagten Radiographie-Systems ist;
ii) die Bestimmung im Volumen V1 eines Zentrums des Unter-Volumens V2 in Abhängigkeit von der Position (x, y, z) des Supports (105); und,
iii) die Berechnung und Rekonstruktion des Unter-Volumens V2 ausgehend vom Volumen V1 in einer Rekonstruktionsachse, die parallel zur Lichteinfallachse verläuft.

9. Verfahren nach einen der Ansprüche 3 bis 8, **dadurch gekennzeichnet, dass** das Unter-Volumen V2 Abmessungen n_{x'} mal n_{y'} mal n_{z'} aufweist, die von einem Bediener festgelegt werden.

10. Verfahren nach einem der vorherigen Ansprüche, **dadurch gekennzeichnet, dass** der Schritt a) die folgenden Unter-Schritte umfasst:
- a1) die Erfassung einer Reihe von Schnitten der Region von Bedeutung; und,
- a2) die Rekonstruktion des Volumens V1 in Form einer dreidimensionalen Voxel-Matrix.

11. Verfahren nach einem der Ansprüche 1 bis 10, **dadurch gekennzeichnet, dass** der Schritt c) die folgenden Unter-Schritte umfasst:
c1) das Lesen des Röntgenaüfnahmebildes IS1 in den Speichervorrichtungen des Radiographic-Systems,
c2) die Überlagerung oder die Subtraktion vom Projektionsbild (IP, IP3) und/ oder des unter-Volumens in einem bestimmten ebenen Schnitt des Röntqenaufnahmebildes IS1.

12. Radiographie-System, das in der Art gestaltet ist, dass es eine Röntgenstrahlenquelle und Speichervorrichtungen umfasst, die gegenüber der besagten Quelle angeordnet sind, sowie einen Support, auf dem ein zu röntgender Gegenstand umfassend die Region von Bedeutung angeordnet werden soll, Vorrichtungen zum Erfassen von dreidimensionalen Daten, die mit den Speichervorrichtungen verbunden sind, Berechnungsverrichtungen und Anzeigevorrichtungen, **dadurch gekennzeichnet, dass** alle diese Vorrichtungen so angeordnet sind, dass sie das Verfahren nach einem der vorherigen Ansprüche anwenden.
